# EUROPÄISCHE PATENTANMELDUNG

(11) **EP 3 772 540 A1**
(43) Veröffentlichungstag der Anmeldung: **10.02.2021**
(21) Anmeldenummer: 19190734.4
(22) Anmeldetag: 08.08.2019
(51) Int. Cl.: C12N 9/18, C12N 15/63

(54) **LIPASEN MIT ERHÖHTER THERMOSTABILITÄT**

(71) Anmelder: Henkel AG & Co. KGaA, 40589 Düsseldorf (DE)
(72) Erfinder: MUSSMANN, Nina, 47877 Willich (DE); WIELAND, Susanne, 41541 Zons/Dormagen (DE); DEGERING, Christian, 40699 Erkrath (DE); PRUESER, Inken, 40215 Düsseldorf (DE); WEIS, Roland, 8062 Kumberg (AT); JOOSTEN, Henk-Jan, 6536CG Nijmegen (NL)

(57) **Zusammenfassung**

Die Erfindung betrifft Lipasen umfassend eine Aminosäuresequenz, die mindestens 70% Sequenzidentität mit der in SEQ ID NO:1 angegebenen Aminosäuresequenz über deren Gesamtlänge aufweist und die Aminosäuresubstitutionen an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295, jeweils bezogen auf die Nummerierung gemäß SEQ ID NO:1, aufweist, besonders bevorzugt sind die mindestens vier Aminosäuresubstitutionen aus der Gruppe bestehend aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F, jeweils bezogen auf die Nummerierung gemäß SEQ ID NO:1, ausgewählt. Die Erfindung betrifft ferner entsprechende Herstellungsverfahren, Wasch- und Reinigungsmittel enthaltend solche Lipasen und entsprechende Verwendungen solcher Lipasen.

## Beschreibung

Die Erfindung liegt auf dem Gebiet der Enzymtechnologie. Die Erfindung betrifft Lipasen aus *Rhizopus oryzae,* deren Aminosäuresequenz, insbesondere im Hinblick auf den Einsatz in Wasch- und Reinigungsmitteln verändert wurde, um ihnen eine bessere Thermostabilität zu verleihen, und die für sie codierende Nukleinsäuren sowie deren Herstellung. Die Erfindung betrifft ferner die Verwendungen dieser Lipasen und Verfahren, in denen sie eingesetzt werden, sowie diese enthaltende Mittel, insbesondere Wasch- und Reinigungsmittel.

Lipasen gehören zu den technisch bedeutendsten Enzymen überhaupt. Ihr Einsatz in Wasch- und Reinigungsmittel ist industriell etabliert und sie sind in praktisch allen modernen, leistungsfähigen Wasch- und Reinigungsmitteln enthalten. Lipasen sind Enzyme, die die Hydrolyse von Esterbindungen in Lipidsubstraten, insbesondere in Fetten und Ölen, katalysieren und damit zur Gruppe der Esterasen gehören. Lipasen sind typischerweise Enzyme, die eine Vielzahl an Substraten spalten können, z.B. aliphatische, alizyklische, bizyklische und aromatische Ester, Thioester und aktivierte Amine. Lipasen werden zur Entfernung von fetthaltigen Anschmutzungen eingesetzt, indem sie deren Hydrolyse (Lipolyse) katalysieren. Lipasen mit breiten Substratspektren werden insbesondere dort verwendet, wo inhomogene Rohstoffe oder Substratgemische umgesetzt werden müssen, also z.B. in Wasch- und Reinigungsmitteln, da Anschmutzungen aus unterschiedlich aufgebauten Fetten und Ölen bestehen können. Die in den aus dem Stand der Technik bekannten Wasch- oder Reinigungsmitteln eingesetzten Lipasen sind üblicherweise mikrobiellen Ursprungs und stammen in der Regel aus Bakterien oder Pilzen, z.B. der Gattungen *Bacillus, Pseudomonas, Acinetobacter, Micrococcus, Humicola, Trichoderma* oder *Trichosporon.* Lipasen werden üblicherweise nach an sich bekannten biotechnologischen Verfahren durch geeignete Mikroorganismen produziert, z.B. durch transgene Expressionswirte der Gattungen *Bacillus* oder durch filamentöse Pilze.

In EP0443063 ist z.B. eine für Wasch- und Reinigungsmittel vorgesehene Lipase aus *Pseudomonas sp.* ATCC 21808 offenbart. In JP1225490A, WO2017097590 und WO2018145927 sind eine Lipase aus *Rhizopus oryzae* bzw. Varianten davon offenbart.

Generell sind nur ausgewählte Lipasen für den Einsatz in flüssigen tensidhaltigen Zubereitungen überhaupt geeignet. Viele Lipasen zeigen in derartigen Zubereitungen keine ausreichende katalytische Leistung oder Stabilität. Insbesondere in Waschverfahren, die im Allgemeinen bei Temperaturen durchgeführt werden, die höher als 20°C liegen, zeigen viele Lipasen thermische Instabilität, die wiederum zu einer unzureichenden katalytischen Aktivität während des Waschvorgangs führt. In phosphonathaltigen flüssigen Tensidzubereitungen ist diese Problematik noch gravierender, z.B. auf Grund der komplexbildenden Eigenschaften der Phosphonate oder auf Grund von unvorteilhaften Wechselwirkungen zwischen dem Phosphonat und der Lipase.

Folglich haben lipase- und tensidhaltige flüssige Formulierungen aus dem Stand der Technik den Nachteil, dass sie oftmals in den Temperaturbereichen, die ein Waschverfahren erfordert, keine zufriedenstellende lipolytische Aktivität aufweisen und daher keine optimale Reinigungsleistung an Lipase-sensitiven Anschmutzungen zeigen.

Überraschenderweise wurde jetzt festgestellt, dass eine Lipase aus *Rhizopus oryzae* oder eine hierzu hinreichend ähnliche Lipase (bezogen auf die Sequenzidentität), die Aminosäuresubstitutionen an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295, jeweils bezogen auf die Nummerierung gemäß SEQ ID NO:1, aufweist, hinsichtlich der (thermischen) Stabilität gegenüber der Wildtypform bzw. der jeweiligen Ausgangsvariante verbessert ist und daher besonders für den Einsatz in Wasch- oder Reinigungsmitteln geeignet ist.

Gegenstand der Erfindung ist daher in einem ersten Aspekt eine Lipase umfassend eine Aminosäuresequenz, die mindestens 70% Sequenzidentität mit der in SEQ ID NO:1 angegebenen Aminosäuresequenz über deren Gesamtlänge aufweist und die Aminosäuresubstitutionen an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295, jeweils bezogen auf die Nummerierung gemäß SEQ ID NO:1, aufweist.

Bevorzugt sind die mindestens vier Aminosäuresubstitutionen aus der Gruppe bestehend aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F, jeweils bezogen auf die Nummerierung gemäß SEQ ID NO:1, ausgewählt.

Weiter bevorzugt weist die Lipase die Aminosäuresubstitutionen N124E, H229N und S295F, jeweils bezogen auf die Nummerierung gemäß SEQ ID NO:1, und mindestens eine weitere Aminosäuresubstitution an einer der Positionen von 19, 23, 37, 41, 82, 101, 103, 116, 117, 129, 132, 138, 142, 148, 153, 156, 160, 161, 183, 190, 197, 223, 239, 256 oder 290, jeweils bezogen auf die Nummerierung gemäß SEQ ID NO:1, auf. Bevorzugt ist die mindestens eine weitere Aminosäuresubstitution aus der Gruppe bestehend aus I19T, S23T, A37L, Q41L, T82Y, Q101R, T103P, S116N, A117S, K129D, K132P, A138S, S142D, V148I, P153H, Q156H, T160D, A161Q, G183A, E190D, N197I, P223S, P239Q, P239A, P239D, P239K, P239S, P239Y, G256P, D290G, D290E oder D290C, jeweils bezogen auf die Nummerierung gemäß SEQ ID NO:1, ausgewählt. Ganz besonders bevorzugt weist die Lipase eine der folgenden Aminosäuresubstitutionsvarianten auf: N124E + H229N + S295F + S23T + P223S + A117S; N124E + H229N + S295F + V148I; N124E + H229N + S295F + P239Q + Q156H; N124E + H229N + S295F + P153H + P239A; N124E + H229N + S295F + I19T + E190D; N124E + H229N + S295F + P153H + P223S + P239A; N124E + H229N + S295F + S23T + P153H + P223S + P239A; N124E + H229N + S295F + P153H + P239Q; N124E + H229N + S295F + P153H + P239A + D290G; N124E + H229N + S295F + P153H + P223S + P239A + D290G + A117S + E190D; N124E + H229N + S295F + S23T + P153H + P223S + P239A + D290G; N124E + H229N + S295F + S23T + P153H + P223S + P239D; N124E + H229N + S295F + S23T + P153H + P223S + P239K; N124E + H229N + S295F + S23T + P153H + P223S + P239S + D290G; N124E + H229N + S295F + S23T + P153H + P223S + P239K + D290E; N124E + H229N + S295F + S23T + P153H + P223S + P239A + D290C; N124E + H229N + S295F + S23T + P153H + P223S + P239K + D290C; N124E + H229N + S295F + S23T + P153H + P223S + P239Y; N124E + H229N + S295F + S23T + P153H + P223S + P239Q + D290G; N124E + H229N + S295F + S23T + P153H + P223S + P239S + D290G + T82Y + S142D + A161Q + G183A; N124E + H229N + S295F + S23T + P153H + P223S + P239S + D290G + Q101R + A138S + T160D + G256P; N124E + H229N + S295F + S23T + P153H + P223S + P239S + D290G + Q41L + T103P + A138S + N197I; N124E + H229N + S295F + S23T + P153H + P223S + P239S + D290G + A37L + S116N + K132P + N197I; N124E + H229N + S295F + S23T + P153H + P223S + P239S + D290G + K129D + K132P + T160D + G183A, jeweils bezogen auf die Nummerierung gemäß SEQ ID NO:1.

Ein weiterer Gegenstand der Erfindung ist ein Verfahren zur Herstellung einer Lipase umfassend das Substituieren einer Aminosäure an mindestens vier der Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 in SEQ ID NO:1 entsprechen, in einer Ausgangslipase, die mindestens 70% Sequenzidentität zu der in SEQ ID NO:1 angegebenen Aminosäuresequenz über deren Gesamtlänge aufweist, vorzugsweise derart, dass die Lipase an mindestens vier Positionen die Aminosäuresubstitution I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F umfasst.

Bevorzugt ist ein Verfahren zur Herstellung einer Lipase umfassend das Einbringen der Aminosäuresubstitutionen N124E, H229N und S295F in Kombination mit mindestens einer weiteren Aminosäuresubstitution, die aus der aus I19T, S23T, A37L, Q41L, T82Y, Q101R, T103P, S116N, A117S, K129D, K132P, A138S, S142D, V148I, P153H, Q156H, T160D, A161Q, G183A, E190D, N197I, P223S, P239Q, P239A, P239D, P239K, P239S, P239Y, G256P, D290G, D290E und D290C, jeweils bezogen auf die Nummerierung gemäß SEQ ID NO:1, bestehenden Gruppe ausgewählt ist.

Eine Lipase im Sinne der vorliegenden Patentanmeldung umfasst daher sowohl die Lipase als solche als auch eine mit einem erfindungsgemäßen Verfahren hergestellte Lipase. Alle Ausführungen zur Lipase beziehen sich daher sowohl auf die Lipase als solche wie auch auf die mittels entsprechender Verfahren hergestellten Lipasen.

Durch bevorzugte Ausführungsformen der vorliegenden Erfindung werden Wasch- und Reinigungsmittel mit verbesserter enzymbasierter (lipolytischer) Reinigungsleistung bereitgestellt. Bevorzugte Ausführungsformen erfindungsgemäßer Lipasen erzielen solche vorteilhaften Reinigungsleistungen auch schon bei niedrigen Temperaturen, insbesondere in den Temperaturbereichen zwischen 10 und 60°C, bevorzugt zwischen 15 und 50°C und besonders bevorzugt zwischen 20 und 40°C. Weitere bevorzugte Ausführungsformen erfindungsgemäßer Lipasen weisen eine verbesserte Thermostabilität und/oder einen verringerten Schlechtgeruch und/oder eine verbesserte Reinigungsleistung rauf.

Weitere Aspekte der Erfindung betreffen die für diese Lipasen codierenden Nukleinsäuren, erfindungsgemäße Lipasen oder Nukleinsäuren enthaltende nicht menschliche Wirtszellen sowie erfindungsgemäße Lipasen umfassende Mittel, insbesondere Wasch- und Reinigungsmittel, Wasch- und Reinigungsverfahren, und Verwendungen der erfindungsgemäßen Lipasen in Wasch- oder Reinigungsmitteln zur Entfernung von fetthaltigen Anschmutzungen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden. "Mindestens eine", wie hierin verwendet, bedeutet eine oder mehrere, d.h. 1, 2, 3, 4, 5, 6, 7, 8, 9, 10, 11, 12, 13, 14 oder mehr. Der Begriff "Wasch- und Reinigungsmittel" bzw. "Wasch- oder Reinigungsmittel", wie hierin verwendet, ist gleichbedeutend mit dem Begriff "Mittel" und bezeichnet eine Zusammensetzung zum Reinigen von Textilien und/oder harten Oberflächen, wie in der Beschreibung erläutert. "Etwa", "ca." oder "ungefähr", wie hierin in Bezug auf einen Zahlenwert verwendet, beziehen sich auf den entsprechenden Zahlenwert ± 10%, vorzugsweise ± 5%.

Die vorliegende Erfindung basiert auf der überraschenden Erkenntnis der Erfinder, dass eine Aminosäuresubstitution an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295 der Lipase aus *Rhizopus oryzae* gemäß SEQ ID NO:1, in einer Lipase, die eine zu der in SEQ ID NO:1 angegebenen Aminosäuresequenz zu mindestens 70% identische Aminosäuresequenz umfasst, insbesondere derart, dass die mindestens vier Aminosäuresubstitutionen aus der Gruppe bestehend aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F, jeweils bezogen auf die Nummerierung gemäß SEQ ID NO:1, ausgewählt sind, eine verbesserte (thermische) Stabilität dieser veränderten Lipase in Wasch- und Reinigungsmitteln bewirkt.

Das ist insbesondere insoweit überraschend, als dass keine der oben genannten Aminosäuresubstitutionen zuvor mit einer erhöhten Stabilität der Lipase in Verbindung gebracht wurde. Die erfindungsgemäßen Lipasen verfügen über eine erhöhte Stabilität in Wasch- oder Reinigungsmitteln, insbesondere gegenüber erhöhten Temperaturen. Solche leistungsverbesserten Lipasen ermöglichen verbesserte Waschergebnisse an lipasesensitiven Anschmutzungen auf Textilien und/oder harten Oberflächen in einem weiten Temperaturbereich.

Die erfindungsgemäßen Lipasen weisen enzymatische Aktivität auf, das heißt, sie sind zur Hydrolyse von Fetten und Ölen befähigt, insbesondere in einem Wasch- oder Reinigungsmittel. Eine erfindungsgemäße Lipase ist daher ein Enzym, welches die Hydrolyse von Esterbindungen in Lipid-Substraten katalysiert und dadurch in der Lage ist, Fette oder Öle zu spalten. Ferner handelt es sich bei einer erfindungsgemäßen Lipase vorzugsweise um eine reife (mature) Lipase, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Lipase ein frei vorliegendes Enzym. Dies bedeutet, dass die Enzyme mit allen Komponenten eines Mittels direkt agieren können und, falls es sich bei dem Mittel um ein Flüssigmittel handelt, dass die Enzyme direkt mit dem Lösungsmittel des Mittels (z.B. Wasser) in Kontakt stehen. In anderen Ausführungsformen kann ein Mittel Enzyme enthalten, die einen Interaktionskomplex mit anderen Molekülen bilden oder die eine "Umhüllung" enthalten. Hierbei kann ein einzelnes oder mehrere Enzymmolekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus sp.,* die die erfindungsgemäßen Enzyme exprimieren, oder Zellkulturüberstände solcher Zellen enthalten.

In verschiedenen Ausführungsformen enthält die erfindungsgemäße Lipase mindestens vier Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F, jeweils bezogen auf die Nummerierung gemäß SEQ ID NO:1, bestehenden Gruppe ausgewählt sind.

In weiter bevorzugten Ausführungsformen weist die Lipase die Aminosäuresubstitutionen N124E, H229N und S295F, jeweils bezogen auf die Nummerierung gemäß SEQ ID NO:1, und mindestens eine weitere Aminosäuresubstitution, die aus der aus I19T, S23T, A37L, Q41L, T82Y, Q101R, T103P, S116N, A117S, K129D, K132P, A138S, S142D, V148I, P153H, Q156H, T160D, A161Q, G183A, E190D, N197I, P223S, P239Q, P239A, P239D, P239K, P239S, P239Y, G256P, D290G, D290E und D290C, jeweils bezogen auf die Nummerierung gemäß SEQ ID NO:1, bestehenden Gruppe ausgewählt ist, auf.

In einer weiteren Ausführungsform der Erfindung umfasst die Lipase eine Aminosäuresequenz, die zu der in SEQ ID NO:1 angegebenen Aminosäuresequenz über deren Gesamtlänge zu mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295 in der Zählung gemäß SEQ ID NO:1 vier oder mehrere der Aminosäuresubstitutionen aufweist, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C und S295F, jeweils bezogen auf die Nummerierung gemäß SEQ ID NO:1, bestehenden Gruppe ausgewählt sind.

In besonders bevorzugten Ausführungsformen enthält die erfindungsgemäße Lipase eine der folgenden Aminosäuresubstitutionsvarianten:
(i) N124E + H229N + S295F + S23T + P223S + A117S;
(ii) N124E + H229N + S295F + V148I;
(iii) N124E + H229N + S295F + P239Q + Q156H;
(iv) N124E + H229N + S295F + P153H + P239A;
(v) N124E + H229N + S295F + I19T + E190D;
(vi) N124E + H229N + S295F + P153H + P223S + P239A;
(vii) N124E + H229N + S295F + S23T + P153H + P223S + P239A;
(viii) N124E + H229N + S295F + P153H + P239Q;
(ix) N124E + H229N + S295F + P153H + P239A + D290G;
(x) N124E + H229N + S295F + P153H + P223S + P239A + D290G + A117S + E190D;
(xi) N124E + H229N + S295F + S23T + P153H + P223S + P239A + D290G;
(xii) N124E + H229N + S295F + S23T + P153H + P223S + P239D;
(xiii) N124E + H229N + S295F + S23T + P153H + P223S + P239K;
(xiv) N124E + H229N + S295F + S23T + P153H + P223S + P239S + D290G;
(xv) N124E + H229N + S295F + S23T + P153H + P223S + P239K + D290E;
(xvi) N124E + H229N + S295F + S23T + P153H + P223S + P239A + D290C;
(xvii) N124E + H229N + S295F + S23T + P153H + P223S + P239K + D290C;
(xviii) N124E + H229N + S295F + S23T + P153H + P223S + P239Y;
(xix) N124E + H229N + S295F + S23T + P153H + P223S + P239Q + D290G;
(xx) N124E + H229N + S295F + S23T + P153H + P223S + P239S + D290G + T82Y + S142D + A161Q + G183A;
(xxi) N124E + H229N + S295F + S23T + P153H + P223S + P239S + D290G + Q101R + A138S + T160D + G256P;
(xxii) N124E + H229N + S295F + S23T + P153H + P223S + P239S + D290G + Q41L + T103P + A138S + N197I;
(xxiii) N124E + H229N + S295F + S23T + P153H + P223S + P239S + D290G + A37L + S116N + K132P + N197I; oder
(xxiv) N124E + H229N + S295F + S23T + P153H + P223S + P239S + D290G + K129D + K132P + T160D + G183A,
wobei die Nummerierung jeweils bezogen auf die Nummerierung gemäß SEQ ID NO:1 ist.

Im Zusammenhang mit der vorliegenden Erfindung bedeutet das Merkmal, dass eine Lipase die angegebenen Substitutionen aufweist, dass sie mindestens eine der entsprechenden Aminosäuren an den entsprechenden Positionen enthält, d.h. nicht alle der genannten Positionen anderweitig mutiert oder, z.B. durch Fragmentierung der Lipase, deletiert sind.

Vorteilhafte Positionen für Sequenzveränderungen, insbesondere Substitutionen, der Lipase aus *Rhizopus oryzae,* die übertragen auf homologe Positionen der erfindungsgemäß eingesetzten Lipasen bevorzugt von Bedeutung sind und der Lipase vorteilhafte funktionelle Eigenschaften verleihen, sind demnach die Positionen, die in einem Alignment den Positionen 19, 23, 37, 41, 82, 101, 103, 116, 117, 124, 129, 132, 138, 142, 148, 153, 156, 160, 161, 183, 190, 197, 223, 229, 239, 256, 290 und 295 in SEQ ID NO:1 entsprechen, d.h. in der Zählung gemäß SEQ ID NO:1. An den genannten Positionen liegen in dem Wildtypmolekül der Lipase aus *Rhizopus oryzae* folgende Aminosäurereste: I19, S23, A37, Q41, T82, Q101, T103, S116, A117, N124, K129, K132, A138, S142, V148, P153, Q156, T160, A161, G183, E190, N197, P223, H229, P239, G256, D290 und S295.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410 und Altschul et al. (1997) "Gapped BLAST and PSI-BLAST: a new generation of protein database search programs", Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen bzw. Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, d.h. dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz. Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

Ein weiterer Gegenstand der vorliegenden Erfindung ist weiter ein Wasch- oder Reinigungsmittel, umfassend mindestens eine Lipase, wobei die Lipase aus einer erfindungsgemäßen Lipase als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen z.B.: G=A=S, I=V=L=M, D=E, N=Q, K=R, Y=F, S=T, G=A=I=V=L=M=Y=F=W=P=S=T.

Ein weiterer Gegenstand der vorliegenden Erfindung ist weiter ein Wasch- oder Reinigungsmittel, umfassend mindestens eine Lipase, wobei die Lipase aus einer erfindungsgemäßen Lipase als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 291, 292, 293, 294, 295, 296 oder 297 zusammenhängenden Aminosäuren mit dem Ausgangsmolekül übereinstimmt.

So ist es z.B. möglich, an den Termini oder in den Loops des Enzyms einzelne Aminosäuren zu deletieren, ohne dass dadurch die enzymatische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre enzymatische Aktivität, d.h. ihre enzymatische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Eine erfindungsgemäße Lipase kann zusätzlich stabilisiert sein, insbesondere durch eine oder mehrere Mutationen, z.B. Substitutionen, oder durch Kopplung an ein Polymer. Denn eine Erhöhung der Stabilität bei der Lagerung und/oder während des Einsatzes, z.B. beim Reinigungsprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Weitere Möglichkeiten der Stabilisierung sind z.B.:
- Veränderung der Bindung von Metallionen, insbesondere der Calcium-Bindungsstellen, z.B. durch Austauschen einer oder mehrerer der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss denaturierender Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Lipase wie vorstehend beschrieben, die dadurch gekennzeichnet ist, dass sie mindestens eine chemische Modifikation aufweist. Eine Lipase mit einer solchen Veränderung wird als Derivat bezeichnet, d.h. die Lipase ist derivatisiert. Unter Derivaten werden im Sinne der vorliegenden Anmeldung demnach solche Proteine verstanden, deren reine Aminosäurekette chemisch modifiziert worden ist. Solche Derivatisierungen können z.B. *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden.

"Variante", wie hierin verwendet, bezieht sich auf natürliche oder artifiziell erzeugte Variationen eines nativen Enzyms, die eine gegenüber der Referenzform abgewandelte Aminosäuresequenz aufweist. Zusätzlich zu den vorstehend erläuterten Aminosäureveränderungen können erfindungsgemäße Enzyme weitere Aminosäureveränderungen, insbesondere Aminosäuresubstitutionen, -insertionen oder -deletionen, aufweisen. Solche Enzyme sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Enzyme oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität des Enzyms erhöht und dadurch seine Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Lipase kann daher im Rahmen der Erfindung zusätzlich weiterentwickelt sein, z.B. hinsichtlich ihrer Stabilität gegenüber Tensiden und/oder Bleichmitteln und/oder anderen Komponenten.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche, Kommata oder Pluszeichen voneinander getrennt. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P14H die Substitution von Prolin an Position 14 durch Histidin, P14HT die Insertion von Threonin nach der Aminosäure Histidin an Position 14 und P14* oder ΔP14 die Deletion von Prolin an Position 14. Diese Nomenklatur ist dem Fachmann auf dem Gebiet der Enzymtechnologie bekannt.

Die Aminosäurepositionen werden durch ein Alignment der Aminosäuresequenz einer erfindungsgemäß eingesetzten Lipase mit der Aminosäuresequenz der Lipase aus *Rhizopus oryzae,* wie sie in SEQ ID NO:1 angegeben ist, definiert. Weiterhin richtet sich die Zuordnung der Positionen nach dem reifen (maturen) Protein. Diese Zuordnung ist insbesondere auch dann anzuwenden, wenn die Aminosäuresequenz einer erfindungsgemäß eingesetzten Lipase eine höhere Zahl von Aminosäureresten umfasst als die Lipase aus *Rhizopus oryzae* gemäß SEQ ID NO:1. Ausgehend von den genannten Positionen in der Aminosäuresequenz der Lipase aus *Rhizopus oryzae* sind die Veränderungspositionen in einer erfindungsgemäß eingesetzten Lipase diejenigen, die eben diesen Positionen in einem Alignment zugeordnet sind.

In einer weiteren Ausführungsform der Erfindung ist die Lipase dadurch gekennzeichnet, dass ihre Reinigungsleistung gegenüber derjenigen einer Lipase, die eine Aminosäuresequenz umfasst, die der in SEQ ID NO:1 angegebenen Aminosäuresequenz entspricht, nicht signifikant verringert ist, d.h. mindestens 80% der Referenzwaschleistung besitzt, vorzugsweise mindestens 100%, weiter bevorzugt mindestens 110%, noch weiter bevorzugt mindestens 120% oder mehr.

Unter Wasch- bzw. Reinigungsleistung wird das Vermögen eines Wasch- oder Reinigungsmittels, eine vorhandene Anschmutzung teilweise oder vollständig zu entfernen, verstanden. Im Rahmen der Erfindung weisen sowohl das Wasch- oder Reinigungsmittel, welches die erfindungsgemäße Lipase umfasst, bzw. die durch dieses Mittel gebildete Wasch- bzw. Reinigungsflotte, als auch die Lipase selbst eine jeweilige Reinigungsleistung auf. Die Reinigungsleistung des Enzyms trägt somit zur Reinigungsleistung des Mittels bzw. der durch das Mittel gebildeten Wasch- bzw. Reinigungsflotte bei.

Unter Wasch- bzw. Reinigungsflotte wird diejenige das Wasch- oder Reinigungsmittel enthaltende Gebrauchslösung verstanden, die auf die Textilien bzw. harten Oberflächen einwirkt und damit mit den auf den Textilien bzw. harten Oberflächen vorhandenen Anschmutzungen in Kontakt kommt. Üblicherweise entsteht die Wasch- bzw. Reinigungsflotte, wenn der Wasch- bzw. Reinigungsvorgang beginnt und das Wasch- oder Reinigungsmittel z.B. in einer Wasch- oder Spülmaschine oder in einem anderen geeigneten Behältnis mit Wasser verdünnt wird.

Die Reinigungsleistung wird in einem Waschsystem bestimmt, das ein Waschmittel in einer Dosierung zwischen 3,5 und 7,0 Gramm pro Liter Waschflotte sowie die Lipase enthält. Die zu vergleichenden Lipasen werden konzentrationsgleich (bezogen auf aktives Protein) eingesetzt. Die Reinigungsleistung der Lipase wird gegenüber lipasesensitiven Anschmutzungen durch Messung des Reinigungsgrades der gewaschenen Textilien bestimmt. Der Waschvorgang erfolgt für 70 Minuten bei einer Temperatur von 40°C, wobei das Wasser eine Wasserhärte zwischen 13,5 und 16,5° (deutsche Härte) aufweist. Die Konzentration der Lipase in dem für dieses Waschsystem bestimmten Waschmittel beträgt 0,001 bis 0,1 Gew.-%, vorzugsweise 0,01 bis 0,06 Gew.-%, bezogen auf aktives Protein.

Ein bevorzugtes flüssiges Waschmittel für ein solches Waschsystem ist wie folgt zusammengesetzt (alle Angaben in Gewichts-Prozent):0,5-3% Glycerin, 0-11% 1,2-Propylemglykol, 2-9% FAEOS (Fettalkoholethersulfat), 3-15% nichtionische Tenside, 3-15% anionische Tenside (LAS), 0,3-1% Borsäure, 0,2-3% Natriumcitrat (Dihydrat), 0,2-3% NaOH, 0,4-4% Kokosnuss-Fettsäuren, 0,1-2,5% HEDP (1-Hydroxyethan-(1,1-di-phosphonsäure)) oder DTPMP, 0-7% Monoethanolamin, 0-0,4% PVP (Polyvinylpyrrolidon), 0-0,15% optischer Aufheller, 0-0,001% Farbstoff, Rest demineralisiertes Wasser. Bevorzugt beträgt die Dosierung des flüssigen Waschmittels zwischen 2,5 und 5,0 Gramm pro Liter Waschflotte bzw. etwa 55 g/Job. Bevorzugt wird gewaschen in einem pH-Wertebereich zwischen pH 7 und pH 10,5, bevorzugt zwischen pH 7,5 und pH 8,5.

Ein bevorzugtes pulverförmiges Waschmittel für ein solches Waschsystem ist wie folgt zusammengesetzt (alle Angaben in Gewichts-Prozent): 10-15% lineares Alkylbenzolsulfonat (Natrium-Salz), 0-1,5% C₁₂₋₁₈-Fettalkoholsulfat (Natrium-Salz), 2-5% C₁₂₋₁₈-Fettalkohol mit 7 EO, 0-2% Seifen, 5-20% Natriumcarbonat, 4-10% amorphes Natriumdisilikat, 0,5-2% Phosphonat (z.B. HEDP-Na4), 1-4%% Polyacrylat, 1-2% Carboxymethylcellulose, Rest: Natriumsulfat, Schauminhibitoren, optischer Aufheller, Duftstoffe, etc. Bevorzugt beträgt die Dosierung des pulverförmigen Waschmittels zwischen 4,5 und 7,0 Gramm pro Liter Waschflotte, z.B. und besonders bevorzugt 4,7 Gramm pro Liter Waschflotte, oder 5,5, 5,9 oder 6,7 Gramm pro Liter Waschflotte bzw. etwa 65 g/Job. Bevorzugt wird gewaschen in einem pH-Wertebereich zwischen pH 9 und pH 11.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist z.B. das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Enzyme wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Verfahren zur Bestimmung der Lipaseaktivität sind dem Fachmann auf dem Gebiet der Enzymtechnologie geläufig und werden von ihm routinemäßig angewendet (vgl. z.B. Bruno Stellmach, "Bestimmungsmethoden Enzyme für Pharmazie, Lebensmittelchemie, Technik, Biochemie, Biologie, Medizin", Steinkopff Verlag Darmstadt, 1988, S. 172ff). Hierbei werden lipasehaltige Proben zu einer Olivenölemulsion in emulgatorhaltigem Wasser gegeben und bei 30°C und pH 9,0 inkubiert. Dabei werden Fettsäuren freigesetzt. Diese werden mit einem Autotitrator über 20 Minuten laufend mit 0,01 N Natronlauge titriert, so dass der pH-Wert konstant bleibt ("pH-stat-Titration"). Anhand des Natronlauge-Verbrauchs erfolgt mittels Bezugs auf eine Referenzlipaseprobe die Bestimmung der Lipaseaktivität. Ein alternativer Test zur Feststellung der lipolytischen Aktivität der erfindungsgemäßen Lipasen ist ein optisches Messverfahren, bevorzugt ein photometrisches Verfahren. Der hierfür geeignete Test umfasst die lipaseabhängige Spaltung des Substrats para-Nitrophenolbutyrats (pNP-Butyrat). Dieses wird durch die Lipase in para-Nitrophenolat und Butyrat gespalten. Die Anwesenheit von para-Nitrophenolat kann unter Verwendung eines Photometers, z.B. des Tecan Sunrise Geräts und der XFLUOR Software, bei 405 nm ermittelt werden und ermöglicht somit einen Rückschluss auf die enzymatische Aktivität der Lipase.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al., 1948, J. Biol. Chem., 177:751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (Bender et al., 1966, J. Am. Chem. Soc. 88(24):5890-5913) erfolgen.

Alle genannten Sachverhalte sind auch auf die erfindungsgemäßen Verfahren zur Herstellung einer Lipase anwendbar. Demnach umfasst ein erfindungsgemäßes Verfahren ferner einen oder mehrere der folgenden Verfahrensschritte:
(a) Einbringen einer ein- oder mehrfachen konservativen Aminosäuresubstitution, wobei die Lipase mindestens vier der Aminosäuresubstitutionen I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, umfasst;
(b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 361, 362, 363, 364 oder 365 zusammenhängenden Aminosäuren mit dem Ausgangsmolekül übereinstimmt, wobei die Lipase mindestens vier der Aminosäuresubstitutionen I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, umfasst.

Sämtliche Ausführungen gelten auch für die erfindungsgemäßen Verfahren.

In weiteren Ausgestaltungen der Erfindung ist die Lipase bzw. die mit einem erfindungsgemäßen Verfahren hergestellte Lipase noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% identisch zu der in SEQ ID NO:1 angegebenen Aminosäuresequenz über deren Gesamtlänge. Die Lipase bzw. die mit einem erfindungsgemäßen Verfahren hergestellte Lipase weist eine Aminosäuresubstitution an mindestens I19, S23, A37, Q41, T82, Q101, T103, S116, A117, N124, K129, K132, A138, S142, V148, P153, Q156, T160, A161, G183, E190, N197, P223, H229, P239, G256, D290 oder S295, jeweils bezogen auf die Nummerierung gemäß SEQ ID NO:1, auf. In bevorzugteren Ausführungsformen sind die mindestens vier Aminosäuresubstitutionen aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C oder S295F, jeweils bezogen auf die Nummerierung gemäß SEQ ID NO:1, bestehenden Gruppe ausgewählt. In weiter bevorzugten Ausführungsformen umfasst die Lipase eine der folgenden Aminosäuresubstitutionsvarianten:
(i) N124E + H229N + S295F + S23T + P223S + A117S;
(ii) N124E + H229N + S295F + V148I;
(iii) N124E + H229N + S295F + P239Q + Q156H;
(iv) N124E + H229N + S295F + P153H + P239A;
(v) N124E + H229N + S295F + I19T + E190D;
(vi) N124E + H229N + S295F + P153H + P223S + P239A;
(vii) N124E + H229N + S295F + S23T + P153H + P223S + P239A;
(viii) N124E + H229N + S295F + P153H + P239Q;
(ix) N124E + H229N + S295F + P153H + P239A + D290G;
(x) N124E + H229N + S295F + P153H + P223S + P239A + D290G + A117S + E190D;
(xi) N124E + H229N + S295F + S23T + P153H + P223S + P239A + D290G;
(xii) N124E + H229N + S295F + S23T + P153H + P223S + P239D;
(xiii) N124E + H229N + S295F + S23T + P153H + P223S + P239K;
(xiv) N124E + H229N + S295F + S23T + P153H + P223S + P239S + D290G;
(xv) N124E + H229N + S295F + S23T + P153H + P223S + P239K + D290E;
(xvi) N124E + H229N + S295F + S23T + P153H + P223S + P239A + D290C;
(xvii) N124E + H229N + S295F + S23T + P153H + P223S + P239K + D290C;
(xviii) N124E + H229N + S295F + S23T + P153H + P223S + P239Y;
(xix) N124E + H229N + S295F + S23T + P153H + P223S + P239Q + D290G;
(xx) N124E + H229N + S295F + S23T + P153H + P223S + P239S + D290G + T82Y + S142D + A161Q + G183A;
(xxi) N124E + H229N + S295F + S23T + P153H + P223S + P239S + D290G + Q101R + A138S + T160D + G256P;
(xxii) N124E + H229N + S295F + S23T + P153H + P223S + P239S + D290G + Q41L + T103P + A138S + N197I;
(xxiii) N124E + H229N + S295F + S23T + P153H + P223S + P239S + D290G + A37L + S116N + K132P + N197I; oder
(xxiv) N124E + H229N + S295F + S23T + P153H + P223S + P239S + D290G + K129D + K132P + T160D + G183A.

Ein weiterer Gegenstand der Erfindung ist eine Nukleinsäure, die für eine erfindungsgemäße Lipase codiert, sowie ein Vektor enthaltend eine solche Nukleinsäure, insbesondere ein Klonierungsvektor oder ein Expressionsvektor. Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand miteingeschlossen, die eine der vorstehend beschriebenen Lipasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codon(s) durch synonyme Codons ersetzt sein. Dieser Aspekt bezieht sich insbesondere auf die heterologe Expression der erfindungsgemäßen Enzyme. So besitzt jeder Organismus, z.B. eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie z.B. die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind z.B. aus Sambrook et al. (2001) "Molecular cloning: a laboratory manual", 3rd Edition, Cold Spring Laboratory Press, bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen z.B. solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, z.B. durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Lipase beinhaltet, insbesondere eine, die die Lipase in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was z.B. die Transformation mit der Nukleinsäure oder dem Vektor und dessen stabile Etablierung angeht, z.B. einzellige Pilze oder Bakterien. Ferner zeichnen sich bevorzugte Wirtszellen durch eine gute mikrobiologische und biotechnologische Handhabbarkeit aus. Das betrifft z.B. leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Lipasen von den sie produzierenden Zellen nach deren Herstellung modifiziert werden, z.B. durch Anknüpfung von Zuckermolekülen, Formylierungen, Aminierungen, usw. Solche posttranslationalen Modifikationen können die Lipase funktionell beeinflussen.

Weitere bevorzugte Ausführungsformen stellen solche Wirtszellen dar, die aufgrund genetischer Regulationselemente, die z.B. auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein. Bei gramnegativen Bakterien wie z.B. *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie z.B. *Bacilli* oder *Actinomyceten* oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist. Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar. In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie *Actinomyceten* oder Hefen wie *Saccharomyces* oder *Kluyveromyces.* Dies kann z.B. dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotische Systeme besonders im Zusammenhang mit der Proteinsynthese durchführen, gehören z.B. die Bindung niedermolekularer Verbindungen wie Membrananker oder Oligosaccharide. Derartige Oligosaccharid-Modifikationen können z.B. zur Senkung der Allergenizität eines exprimierten Proteins wünschenswert sein. Auch eine Co-Expression mit den natürlicherweise von derartigen Zellen gebildeten Enzymen, wie z.B. Cellulasen, kann vorteilhaft sein. Ferner können sich z.B. thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, z.B. in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann. Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Erfindungsgemäße Wirtszellen werden bevorzugt verwendet, um erfindungsgemäße Lipasen herzustellen. Ein weiterer Gegenstand der Erfindung ist daher ein Verfahren zur Herstellung einer Lipase umfassend
(a) Kultivieren einer erfindungsgemäßen Wirtszelle, und
(b) Isolieren der Lipase aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, z.B. der erfindungsgemäßen Lipasen. Alle Fermentationsverfahren, die auf einem entsprechenden Verfahren zur Herstellung einer erfindungsgemäßen Lipase beruhen, stellen Ausführungsformen dieses Erfindungsgegenstandes dar. Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse und/oder insbesondere in der Aktivität der interessierenden Lipase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden. Die hergestellte Lipase kann aus dem Fermentationsmedium geerntet werden. Ein solches Fermentationsverfahren ist gegenüber einer Isolation der Lipase aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Lipase in das Fermentationsmedium sezernieren. Ohne Sekretion kann alternativ die Isolation der Lipase aus der Wirtszelle, d.h. eine Aufreinigung derselben aus der Zellmasse, erfolgen, z.B. durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Lipasen herzustellen.

Ein weiterer Gegenstand der Erfindung ist ein Mittel, das dadurch gekennzeichnet ist, dass es eine erfindungsgemäße Lipase wie vorstehend beschrieben enthält. Bevorzugt ist das Mittel als ein Wasch- oder Reinigungsmittel.

Unter einem Wasch- oder Reinigungsmittel sind erfindungsgemäß alle denkbaren Wasch- oder Reinigungsmittelarten zu verstehen, sowohl Konzentrate als auch unverdünnt anzuwendende Mittel, zum Einsatz im kommerziellen Maßstab, in der Waschmaschine oder bei der Handwäsche bzw. -reinigung. Dazu gehören z.B. Waschmittel für Textilien, Teppiche, oder Naturfasern, für die die Bezeichnung Waschmittel verwendet wird. Dazu gehören z.B. auch Geschirrspülmittel für Geschirrspülmaschinen (maschinelle Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln z.B. auch Scheuermittel, Glasreiniger, WC-Duftspüler, usw. Zu den Wasch- und Reinigungsmitteln im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu den Wasch- und Reinigungsmitteln im Rahmen der Erfindung auch Textilvor- und Nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Enzymkombination aus Protease und Amylase alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Polymere, Glaskorrosionsinhibitoren, Korrosionsinhibitoren, Bleichmittel wie Persauerstoffverbindungen, Bleichaktivatoren oder Bleichkatalysatoren enthalten. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten.

Vorteilhafte Inhaltsstoffe erfindungsgemäßer Mittel sind offenbart in WO2009121725, dort beginnend auf Seite 5, vorletzter Absatz, und endend auf Seite 13 nach dem zweiten Absatz, sowie in WO2012084582, Seiten 12-27. Auf diese Offenbarung wird ausdrücklich Bezug genommen und der dortige Offenbarungsgehalt in die vorliegende Patentanmeldung einbezogen. Insbesondere können die hierin beschriebenen Lipasen in vorteilhafter Weise mit Phosphonaten kombiniert werden, wie sie in der WO2012084582, Seite 4, zweiter Absatz bis Seite 5, erster Absatz, beschrieben sind.

Ein erfindungsgemäßes Mittel enthält die Lipase vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. Ferner kann die in dem Mittel enthaltene Lipase, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Lipase mit einer bei Raumtemperatur oder bei Abwesenheit von Wasser für die Lipase undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

Die Ausführungsformen der vorliegenden Erfindung umfassen alle festen, pulverförmigen, granularen, tablettenförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 bis 1200 g/l, insbesondere 500 bis 900 g/l oder 600 bis 850 g/l. Zu den festen Darreichungsformen des Mittels zählen ferner Extrudate, Granulate, Tabletten oder Pouches enthaltend feste Mittel, die sowohl in Großgebinden als auch portionsweise abgepackt vorliegen können. Alternativ kann das Mittel auch flüssig, gelförmig oder pastös sein, z.B. in Form eines nicht-wässrigen Mittels oder einer nicht-wässrigen Paste oder in Form eines wässrigen Mittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen (Mehrkomponentensystem). Ein solches Mittel ist demnach in mehrere Komponenten aufgeteilt, z.B. zwei flüssige, zwei feste oder eine flüssige und eine feste Phase. In einer weiteren Ausführungsform der Erfindung ist das Wasch- oder Reinigungsmittel daher dadurch gekennzeichnet sind, dass es in mehrere Komponenten aufgeteilt ist. Die flüssige Angebotsform auf Basis von Wasser und/oder organischen Lösungsmitteln können verdickt, in Form von Gelen vorliegen.

Ein Stoff, z.B. eine Zusammensetzung oder ein Mittel ist gemäß Definition der Erfindung festförmig, wenn sie bei 25°C und 1013 mbar im festen Aggregatzustand vorliegt.

Ein Stoff, z.B. eine Zusammensetzung oder ein Mittel ist gemäß Definition der Erfindung flüssig, wenn sie bei 25°C und 1013 mbar im flüssigen Aggregatzustand vorliegt. Dabei umfasst flüssig auch gelförmig.

Die erfindungsgemäßen Mittel liegen vorzugsweise in flüssiger Form vor. Bevorzugte Wasch- und Reinigungsmittel enthalten bezogen auf ihr Gesamtgewicht mehr als 40 Gew.-%, vorzugsweise zwischen 50 und 90 Gew.-% und insbesondere zwischen 60 und 80 Gew.-% Wasser.

Erfindungsgemäße Wasch- oder Reinigungsmittel können ausschließlich eine Lipase enthalten. Alternativ können sie auch weitere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Lipasen unterscheidbare - Lipasen, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Lipase und einem weiteren Enzym eines erfindungsgemäßen Mittels, darunter insbesondere zwischen der genannten Lipase und einer Amylase und/oder einer Protease und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien und/oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Lipase katalytisch aktiv wird, insbesondere derart, dass die Lipase in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

In verschieden Ausführungsformen zeichnet sich das oben beschriebene Verfahren dadurch aus, dass die Lipase bei einer Temperatur von 0 bis100°C, bevorzugt 10 bis 70°C, weiter bevorzugt 30 bis 50°C, noch weiter bevorzugt 20 bis 40°C und am meisten bei ungefähr 40°C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Lipase bereichert werden und stellen dann Ausführungsformen der vorliegenden Erfindung dar.

Da erfindungsgemäße Lipasen natürlicherweise bereits eine hydrolytische Aktivität besitzen und diese auch in Medien entfalten, die sonst keine Reinigungskraft besitzen wie z.B. in bloßem Puffer, kann ein einzelner und/oder der einzige Schritt eines solchen Verfahrens darin bestehen, dass als einzige reinigungsaktive Komponente eine erfindungsgemäße Lipase mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Lipase aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Lipasen in Wasch- oder Reinigungsmitteln, z.B. wie oben beschrieben, zur (verbesserten) Entfernung von fetthaltigen Anschmutzungen, z.B. von Textilien oder harten Oberflächen.

Alle Sachverhalte, Gegenstände und Ausführungsformen, die für erfindungsgemäße Lipasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

### Beispiele

### Beispiel 1: Generierung und Charakterisierung der Mutanten

Der mature Teil des Lipase Gens wird mittels Error Prone Mutagenese nach bekannten Methoden mutagenisiert ("GeneMorph II Random Mutagenesis Kit" from Agilent). Die Mutanten Bibliothek wird mit üblichen Methoden in *Pichia pastoris* kloniert. Die Kolonien werden in 96-Well Deep-Well Platten gepickt und nach Induktion für weitere 72 Stunden kultiviert. Nach Zentrifugation wird der Überstand auf Lipaseaktivität untersucht. Es wurden mehrere aufeinanderfolgende Error-Prone Runden durchgeführt, wobei in der ersten Runde mit der Wildtyp Lipase (SEQ ID NO:1) gestartet wurde. Weiterhin wurden einige Mutanten durch gezielte Rekombination, nach üblichen Methoden, erstellt. Die generierten Varianten sind in Tabelle 1 dargestellt.

**Tabelle 1: Übersicht der Varianten (Zählweise gemäß SEQ ID NO:1):**

| **Variante** | **Aminosäuresubstitutionen gegenüber dem Wildtyp (SEQ ID NO:1)** | | | | | | | | | | | |
|---|---|---|---|---|---|---|---|---|---|---|---|---|
| Variante 1 | | H229N | S295F | | | | | | | | | |
| Variante 2 | N124E | H229N | S295F | | | | | | | | | |
| Variante 3 | N124E | H229N | S295F | S23T | | P223S | | | A117S | | | |
| Variante 4 | N124E | H229N | S295F | | | | | | V148I | | | |
| Variante 5 | N124E | H229N | S295F | | | | P239Q | | Q156H | | | |
| Variante 6 | N124E | H229N | S295F | | P153H | | P239A | | | | | |
| Variante 7 | N124E | H229N | S295F | | | | | | I19T | E190D | | |
| Variante 8 | N124E | H229N | S295F | | P153H | P223S | P239A | | | | | |
| Variante 9 | N124E | H229N | S295F | S23T | P153H | P223S | P239A | | | | | |
| Variante 10 | N124E | H229N | S295F | | P153H | | P239Q | | | | | |
| Variante 11 | N124E | H229N | S295F | | P153H | | P239A | D290G | | | | |
| Variante 12 | N124E | H229N | S295F | | P153H | P223S | P239A | D290G | A117S | E190D | | |
| Variante 13 | N124E | H229N | S295F | S23T | P153H | P223S | P239A | D290G | | | | |
| Variante 14 | N124E | H229N | S295F | S23T | P153H | P223S | P239D | | | | | |
| Variante 15 | N124E | H229N | S295F | S23T | P153H | P223S | P239K | | | | | |
| Variante 16 | N124E | H229N | S295F | S23T | P153H | P223S | P239S | D290G | | | | |
| Variante 17 | N124E | H229N | S295F | S23T | P153H | P223S | P239K | D290E | | | | |
| Variante 18 | N124E | H229N | S295F | S23T | P153H | P223S | P239A | D290C | | | | |
| Variante 19 | N124E | H229N | S295F | S23T | P153H | P223S | P239K | D290C | | | | |
| Variante 20 | N124E | H229N | S295F | S23T | P153H | P223S | P239Y | | | | | |
| Variante 21 | N124E | H229N | S295F | S23T | P153H | P223S | P239Q | D290G | | | | |
| Variante 22 | N124E | H229N | S295F | S23T | P153H | P223S | P239S | D290G | T82Y | S142D | A161Q | G183A |
| Variante 23 | N124E | H229N | S295F | S23T | P153H | P223S | P239S | D290G | Q101R | A138S | T160D | G256P |
| Variante 24 | N124E | H229N | S295F | S23T | P153H | P223S | P239S | D290G | Q41L | T103P | A138S | N197I |
| Variante 25 | N124E | H229N | S295F | S23T | P153H | P223S | P239S | D290G | A37L | S116N | K132P | N197I |
| Variante 26 | N124E | H229N | S295F | S23T | P153H | P223S | P239S | D290G | K129D | K132P | T160D | G183A |

### Beispiel 2: Aktivitätsassay

Zur Identifizierung von Varianten mit verbesserter Thermostabilität wurde ein Mikrotiterplattenbasierter Assay unter Verwendung von para-Nitrophenol-Palmitat (p-NPP) als Substrat verwendet. Bei enzymatischer Hydrolyse im wässrigen Medium wurden para-Nitrophenolat und Palmitat freigesetzt und anschließend wurde para-Nitrophenolat durch Absorptionsmessung bei einer Wellenlänge von 405 nm detektiert. P-NPP wird in Form einer Emulsion eingesetzt, vorgelöst wird es in einen wässrigen Puffer gegeben, welcher als Emulgatoren Na-Desoxycholat und alpha-Olefinsulfonat (AOS) enthält.

Bedingungen: pH 8,0, 25°C, 405 nm, Messzeit 120 Sekunden mit 30-Sekunden-Intervallen Probenpuffer, in dem die Lipase-Überstände verdünnt werden: 225 mg/ml Brij35, 9 mM CaCl₂ und 4,7 mg/ml Waschmittelmatrix (Tabelle 2)
Substrat-Arbeitspuffer: 96,7 ml Emulgatorlösung (100 mM Tris-HCl pH 8,0, 6,5 mM Desoxycholat, 1,4 g/L AOS) + 3,3 ml Palmitatlösung (7,8 mM p-NPP in Ethanol gelöst)

Durchführung: In der Mikrotiterplatte 20 µl Probe verdünnt in Puffer vorlegen und die Reaktion durch Zugabe von 200 µl des Substrat-Arbeitspuffers starten, 5 Sekunden schütteln, Kinetik starten.

**Tabelle 2: Flüssige Waschmittelmatrix (handelsüblich, ohne Enzyme, opt. Aufheller, Parfüm und Farbstoffe), die für den Aktivitätstest und Waschtest verwendet wurde:**

| Chemischer Name | Gew.-% Aktivsubstanz in der Formulierung |
|---|---|
| Wasser demin. | Rest |
| Alkylbenzolsulfonsäure | 4,4 |
| Weitere anionische Tenside | 5,6 |
| C₁₂-C₁₈ Fettsäuren Na-Salz | 2,4 |
| Nicht-ionische Tenside | 4,4 |
| Phosphonate | 0,2 |
| Zitronensäure | 1,4 |
| NaOH | 0,95 |
| Entschäumer | 0,01 |
| Glycerin | 2,0 |
| Konservierungsmittel | 0,08 |
| Ethanol | 1,0 |
| Ohne opt. Aufheller, Parfüm, Farbstoff und Enzyme | |
| Dosierung 4,7 g/L | |

### Beispiel 3: Stabilitätstest

Um die Thermostabilität der Mutanten zu testen, wurden die in Probenpuffer verdünnten Lipase-Überstände mit 10% Waschmittelmatrix versetzt und für 20 Stunden bei 30°C inkubiert, bevor der p-NPP Test durchgeführt wurde. Es wurden jeweils mindestens Dreifachbestimmungen durchgeführt. Je höher die gemessene Restaktivität, desto besser die Thermostabilität der Lipase-Variante in Waschmittelmatrix. Es gibt für die verschiedenen Varianten, die in unterschiedlichen Mutagenese- bzw. Rekombinationsrunden generiert wurden unterschiedliche Benchmarks, gegenüber der die jeweilige Restaktivität bestimmt wurde. Dabei bezieht sich Variante 2 auf Variante 1 als Benchmark, die Varianten 3 bis 7 beziehen sich auf Variante 2 als Benchmark, die Varianten 8 bis 12 beziehen sich auf Variante 6 als Benchmark, die Varianten 11 bis 21 beziehen sich auf Variante 9 als Benchmark und die Varianten 22 bis 26 beziehen sich auf Variante 16 als Benchmark. Die Restaktivität wurde bestimmt wie im obigen Beispiel angegeben. Die Ergebnisse sind in Tabelle 3 dargestellt.

**Tabelle 3: Ergebnisse des Thermostabilitätstests**

| **Mutante** | **Restaktivität** |
|---|---|
| Variante 2 | 57% |
| Variante 3 | 60% |
| Variante 4 | 41% |
| Variante 5 | 44% |
| Variante 6 | 55% |
| Variante 7 | 40% |
| Variante 8 | 50% |
| Variante 9 | 57% |
| Variante 10 | 63% |
| Variante 11 | 64% |
| Variante 12 | 52% |
| Variante 13 | 95% |
| Variante 14 | 77% |
| Variante 15 | 85% |
| Variante 16 | 90% |
| Variante 17 | 90% |
| Variante 18 | 76% |
| Variante 19 | 90% |
| Variante 20 | 95% |
| Variante 21 | 80% |
| Variante 22 | 88% |
| Variante 23 | 94% |
| Variante 24 | 86% |
| Variante 25 | 103% |
| Variante 26 | 99% |

Alle hier gezeigten Varianten sind stabiler bei 30°C als ihre jeweilige Ausgangs-Variante. Da sich die jeweiligen Ausgangs-Varianten von Variante 1 bzw. vom Wildtyp ableiten, sind alle hier gezeigten Varianten auch stabiler bei 30°C als der Wildtyp sowie Variante 1.

## Patentansprüche

1. Lipase umfassend eine Aminosäuresequenz, die mindestens 70% Sequenzidentität mit der in SEQ ID NO:1 angegebenen Aminosäuresequenz über deren Gesamtlänge aufweist und die Aminosäuresubstitutionen an mindestens vier der Positionen von 19, 23, 37, 41, 82, 101, 103, 116, 117, 124, 129, 132, 138, 142, 148, 153, 156, 160, 161, 183, 190, 197, 223, 229, 239, 256, 290 oder 295, jeweils bezogen auf die Nummerierung gemäß SEQ ID NO:1, aufweist.

2. Lipase nach Anspruch 1, wobei die mindestens vier Aminosäuresubstitutionen aus der Gruppe bestehend aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N1971, P223S, H229N, P239Q, P239A, P239D, P239K, P239S, P239Y, G256P, D290G, D290E, D290C und S295F, jeweils bezogen auf die Nummerierung gemäß SEQ ID NO:1, ausgewählt sind.

3. Lipase nach Anspruch 1 oder 2, **dadurch gekennzeichnet, dass** sie die Aminosäuresubstitutionen N124E, H229N und S295F, jeweils bezogen auf die Nummerierung gemäß SEQ ID NO:1, und mindestens eine weitere Aminosäuresubstitution an einer der Positionen von 19, 23, 37, 41, 82, 101, 103, 116, 117, 129, 132, 138, 142, 148, 153, 156, 160, 161, 183, 190, 197, 223, 239, 256 oder 290, jeweils bezogen auf die Nummerierung gemäß SEQ ID NO:1, aufweist.

4. Lipase nach einem der Ansprüche 1 bis 3, wobei die Lipase eine der folgenden Aminosäuresubstitutionsvarianten aufweist:
(i) N124E + H229N + S295F + S23T + P223S + A117S
(ii) N124E + H229N + S295F + V148I
(iii) N124E + H229N + S295F + P239Q + Q156H
(iv) N124E + H229N + S295F + P153H + P239A
(v) N124E + H229N + S295F + I19T + E190D
(vi) N124E + H229N + S295F + P153H + P223S + P239A
(vii) N124E + H229N + S295F + S23T + P153H + P223S + P239A
(viii) N124E + H229N + S295F + P153H + P239Q
(ix) N124E + H229N + S295F + P153H + P239A + D290G
(x) N124E + H229N + S295F + P153H + P223S + P239A + D290G + A117S + E190D
(xi) N124E + H229N + S295F + S23T + P153H + P223S + P239A + D290G
(xii) N124E + H229N + S295F + S23T + P153H + P223S + P239D
(xiii) N124E + H229N + S295F + S23T + P153H + P223S + P239K
(xiv) N124E + H229N + S295F + S23T + P153H + P223S + P239S + D290G
(xv) N124E + H229N + S295F + S23T + P153H + P223S + P239K + D290E
(xvi) N124E + H229N + S295F + S23T + P153H + P223S + P239A + D290C
(xvii) N124E + H229N + S295F + S23T + P153H + P223S + P239K + D290C
(xviii) N124E + H229N + S295F + S23T + P153H + P223S + P239Y
(xix) N124E + H229N + S295F + S23T + P153H + P223S + P239Q + D290G
(xx) N124E + H229N + S295F + S23T + P153H + P223S + P239S + D290G + T82Y + S142D + A161Q + G183A
(xxi) N124E + H229N + S295F + S23T + P153H + P223S + P239S + D290G + Q101R + A138S + T160D + G256P
(xxii) N124E + H229N + S295F + S23T + P153H + P223S + P239S + D290G + Q41L + T103P + A138S + N197I
(xxiii) N124E + H229N + S295F + S23T + P153H + P223S + P239S + D290G + A37L + S116N + K132P + N197I
(xxiv) N124E + H229N + S295F + S23T + P153H + P223S + P239S + D290G + K129D + K132P + T160D + G183A

5. Lipase, **dadurch gekennzeichnet, dass**
(a) sie aus einer Lipase nach einem der Ansprüche 1 bis 4 als Ausgangsmolekül erhältlich ist durch ein- oder mehrfache konservative Aminosäuresubstitution, wobei die Lipase mindestens vier Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C und S295F bestehenden Gruppe ausgewählt sind, an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, aufweist; und/oder
(b) sie aus einer Lipase nach einem der Ansprüche 1 bis 4 als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 291, 292, 293, 294, 295, 296 oder 297 zusammenhängenden Aminosäuren mit dem Ausgangsmolekül übereinstimmt, wobei die Lipase mindestens vier Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C und S295F bestehenden Gruppe ausgewählt sind, an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, umfasst.

6. Verfahren zur Herstellung einer Lipase umfassend das Substituieren einer Aminosäure an mindestens vier der Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 in SEQ ID NO:1 entsprechen, in einer Ausgangslipase, die mindestens 70% Sequenzidentität zu der in SEQ ID NO:1 angegebenen Aminosäuresequenz über deren Gesamtlänge aufweist, vorzugsweise derart, dass die Lipase an mindestens vier Positionen Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N1971, P223S, H229N, P239Q, P239A, P239D, P239K, P239S, P239Y, G256P, D290G, D290E, D290C und S295F, jeweils bezogen auf die Nummerierung gemäß SEQ ID NO:1, bestehenden Gruppe ausgewählt sind, umfasst.

7. Verfahren nach Anspruch 6, ferner umfassend einen oder beide der folgenden Verfahrensschritte:
(a) Einbringen einer ein- oder mehrfachen konservativen Aminosäuresubstitution, wobei die Lipase mindestens vier Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C und S295F bestehenden Gruppe ausgewählt sind, an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, umfasst;
(b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 291, 292, 293, 294, 295, 296 oder 297 zusammenhängenden Aminosäuren mit dem Ausgangsmolekül übereinstimmt, wobei die Lipase mindestens vier Aminosäuresubstitutionen, die aus der aus I19T, S23T, A37L, Q41L, T82Y, Q101R, T103P, S116N, A117S, N124E, K129D, K132P, A138S, S142D, V148I, P153H, Q156H, T160D, A161Q, G183A, E190D, N197I, P223S, H229N, P239Q, P239A, P239D, P239K, P239S, P239Y, G256P, D290G, D290E, D290C und S295F bestehenden Gruppe ausgewählt sind, an den Positionen, die den Positionen 19, 23, 37, 41, 82, 101, 103, 116, 117, 124, 129, 132, 138, 142, 148, 153, 156, 160, 161, 183, 190, 197, 223, 229, 239, 256, 290 oder 295 gemäß SEQ ID NO:1 entsprechen, umfasst.

8. Nukleinsäure kodierend für eine Lipase nach einem der Ansprüche 1 bis 5 oder kodierend für eine nach einem Verfahren nach Anspruch 6 oder 7 erhältliche Lipase.

9. Vektor enthaltend eine Nukleinsäure nach Anspruch 8, insbesondere ein Klonierungsvektor oder ein Expressionsvektor.

10. Nicht-menschliche Wirtszelle, die eine Nukleinsäure nach Anspruch 8 oder einen Vektor nach Anspruch 9 beinhaltet, oder die eine Lipase nach einem der Ansprüche 1 bis 5 beinhaltet, oder die eine nach einem Verfahren nach Anspruch 6 oder 7 erhältliche Lipase beinhaltet.

11. Verfahren zur Herstellung einer Lipase umfassend
(a) Kultivieren einer Wirtszelle gemäß Anspruch 10; und
(b) Isolieren der Lipase aus dem Kulturmedium oder aus der Wirtszelle.

12. Mittel, insbesondere ein Wasch- oder Reinigungsmittel, **dadurch gekennzeichnet, dass** es mindestens eine Lipase nach einem der Ansprüche 1 bis 5 oder eine nach einem Verfahren nach Anspruch 6 oder 7 erhältliche Lipase enthält.

13. Verfahren zur Reinigung von Textilien oder harten Oberflächen, **dadurch gekennzeichnet, dass** in mindestens einem Verfahrensschritt ein Mittel nach Anspruch 12 angewendet wird, oder dass in mindestens einem Verfahrensschritt eine Lipase nach einem der Ansprüche 1 bis 5 oder eine nach einem Verfahren nach Anspruch 6 oder 7 erhältliche Lipase angewendet wird.

14. Verwendung einer Lipase nach einem der Ansprüche 1 bis 5 oder einer nach einem Verfahren nach Anspruch 6 oder 7 erhältlichen Lipase in einem Wasch- oder Reinigungsmittel zur Entfernung von fetthaltigen Anschmutzungen.


SEQUENCE LISTING

<110>  Henkel AG & Co. KGaA

<120>  Lipasen mit erhöhter Thermostabilität

<130>  2018P35556EP

<160>  1

<170>  PatentIn version 3.5

<210>  1
<211>  297
<212>  PRT
<213>  Rhizopus oryzae

<400>  1

Asp Asp Asn Leu Val Gly Gly Met Thr Leu Asp Leu Pro Ser Asp Ala
1               5                   10                  15

Pro Pro Ile Ser Leu Ser Ser Ser Thr Asn Ser Ala Ser Asp Gly Gly
            20                  25                  30

Lys Val Val Ala Ala Thr Thr Ala Gln Ile Gln Glu Phe Thr Lys Tyr
        35                  40                  45

Ala Gly Ile Ala Ala Thr Ala Tyr Cys Arg Ser Val Val Pro Gly Asn
    50                  55                  60

Lys Trp Asp Cys Val Gln Cys Gln Lys Trp Val Pro Asp Gly Lys Ile
65                  70                  75                  80

Ile Thr Thr Phe Thr Ser Leu Leu Ser Asp Thr Asn Gly Tyr Val Leu
                85                  90                  95

Arg Ser Asp Lys Gln Lys Thr Ile Tyr Leu Val Phe Arg Gly Thr Asn
            100                 105                 110

Ser Phe Arg Ser Ala Ile Thr Asp Ile Val Phe Asn Phe Ser Asp Tyr
        115                 120                 125

Lys Pro Val Lys Gly Ala Lys Val His Ala Gly Phe Leu Ser Ser Tyr
    130                 135                 140

Glu Gln Val Val Asn Asp Tyr Phe Pro Val Val Gln Glu Gln Leu Thr
145                 150                 155                 160

Ala His Pro Thr Tyr Lys Val Ile Val Thr Gly His Ser Leu Gly Gly
                165                 170                 175

Ala Gln Ala Leu Leu Ala Gly Met Asp Leu Tyr Gln Arg Glu Pro Arg
            180                 185                 190

Leu Ser Pro Lys Asn Leu Ser Ile Phe Thr Val Gly Gly Pro Arg Val
        195                 200                 205

Gly Asn Pro Thr Phe Ala Tyr Tyr Val Glu Ser Thr Gly Ile Pro Phe
    210                 215                 220

Gln Arg Thr Val His Lys Arg Asp Ile Val Pro His Val Pro Pro Gln
225                 230                 235                 240

Ser Phe Gly Phe Leu His Pro Gly Val Glu Ser Trp Ile Lys Ser Gly
                245                 250                 255

Thr Ser Asn Val Gln Ile Cys Thr Ser Glu Ile Glu Thr Lys Asp Cys
            260                 265                 270

Ser Asn Ser Ile Val Pro Phe Thr Ser Ile Leu Asp His Leu Ser Tyr
        275                 280                 285

Phe Asp Ile Asn Glu Gly Ser Cys Leu
    290                 295